# EUROPEAN PATENT SPECIFICATION

(11) **EP 3 132 748 B1**
(45) Date of publication and mention of the grant of the patent: **26.12.2018**
(21) Application number: 15779283.9
(22) Date of filing: 13.03.2015
(51) Int. Cl.: A61B 8/00, A61B 90/00

(54) **PORTABLE ULTRASONIC DIAGNOSTIC DEVICE COMPRISING HEAT DISSIPATION STRUCTURE**
TRAGBARE ULTRASCHALLDIAGNOSEVORRICHTUNG MIT WÄRMEABLEITUNGSSTRUKTUR
DISPOSITIF PORTABLE DE DIAGNOSTIC À ULTRASONS AVEC STRUCTURE DE DISSIPATION DE CHALEUR

(30) Priority: 17.04.2014 KR 20140046103
(43) Date of publication of application: 22.02.2017
(73) Proprietor: Healcerion Co., Ltd., Seoul 151-848 (KR)
(72) Inventor: RYU, Jeong Won, Seoul 137-140 (KR); CHOUNG, You Chan, Seoul 122-807 (KR); CHUNG, Wook Jin, Seoul 135-950 (KR)
(74) Representative: Brann AB
(86) International application number: PCT/KR2015/002440
(87) International publication number: WO 2015/160088

(56) References cited:
- WO-A2-2008/146208
- JP-A- 2012 120 691
- KR-A- 20010 009 325
- KR-A- 20050 028 995
- KR-A- 20130 126 510
- US-A- 5 964 709
- US-A1- 2011 282 211
- US-A1- 2012 150 038

## Description

### [Technical Field]

The present invention relates to a portable ultrasonic diagnostic apparatus comprising a heat dissipation structure, and more particularly, to a portable ultrasonic diagnostic apparatus comprising a heat dissipation structure capable of improving performance of the product by externally dissipating heat generated inside the portable ultrasonic diagnostic apparatus.

### [Background Art]

With noninvasive and nondestructive properties, ultrasonic diagnostic systems are generally used in the medical field to obtain information of the inside of a subject. Since it is possible to provide a high-resolution image of internal organizations of the subject to a doctor with no surgical operations of directly incising and observing the subject, ultrasonic diagnostic systems are very importantly used in the medical field.

That is, ultrasonic diagnostic systems are systems which emit an ultrasonic signal from a body surface of a subject toward a target portion inside the subject, extract information from a reflected ultrasonic signal, and obtain an image of a section of soft tissue or a blood flow in a noninvasive manner.

Compared with other diagnostic imaging apparatuses such as X-ray inspection apparatuses, computerized tomography (CT) scanners, magnetic resonance image (MRI) scanners, and nuclear medicine inspection apparatuses, due to a small size, a low price, displaying in real time, and excellent safety without being exposed to X-rays, etc., ultrasonic diagnostic systems described above are generally used to diagnose a heart, internal organs in an abdominal cavity, urinary systems, and genital organs.

Meanwhile, the ultrasonic diagnostic system includes an ultrasonic diagnostic apparatus for transmitting an ultrasonic signal to a subject and receiving the ultrasonic signal reflected by the subject to obtain an ultrasonic image of the subject.

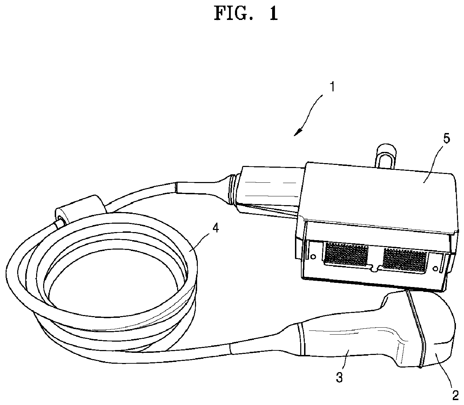

FIG. 1 illustrates a conventional ultrasonic diagnostic apparatus 1 which forms an ultrasonic diagnostic system. The conventional ultrasonic diagnostic apparatus 1 includes a probe body 3 including an ultrasonic probe 2 formed on one end, a cable 4, and a connector 5. The ultrasonic probe 2 includes a piezoelectric element which generates ultrasonic waves and receives echoes therein. The probe body includes a circuit board which generates an ultrasonic image and a power portion which supplies power to the piezoelectric element and the circuit board therein.

However, the conventional ultrasonic diagnostic apparatus 1 described above includes the connector 5 and the cable 4 to be connected to a body of the ultrasonic diagnostic system, a size thereof is too large to carry.

To overcome it, technologies for reducing sizes of conventional ultrasonic diagnostic apparatuses to easily carry and wirelessly transmitting an ultrasonic image to a body of an ultrasonic diagnostic system and an external apparatus have been getting the spotlight.

US 20125/0150038 A1 discloses an apparatus, which can be considered as closest prior art for the invention, namely a portable ultrasonic diagnostic apparatus comprising a heat dissipation structure comprising:
- an external housing which comprises a circuit portion accommodating space accommodating a circuit board inside one side and comprises a battery accommodating portion for accommodating a portable battery therein inside another side;
- a plurality of circuit boards arranged in a vertical stacking structure from top to bottom inside the circuit portion accommodating space of the external housing; and
- a heat conducting pipe with one side disposed between the plurality of circuit boards and another side disposed in the battery accommodating space to absorb heat generated by the plurality of circuit boards and discharge the heat into the outside air.

However, in case of portable ultrasonic diagnostic apparatuses described above, it is difficult to reduce a size of an external housing and a life of a product is reduced by heat generated by a circuit board installed in a limited space.

To overcome limitations described above, it has been tried to install a vent which circulates outside air at a place on which a circuit board is mounted. However, in this case, it is difficult to satisfy waterproof standards needed by portable ultrasonic diagnostic apparatuses.

Accordingly, it is in desperate need of a technology related to a heat dissipation structure of a portable ultrasonic diagnostic apparatus, capable of satisfying necessary waterproof standards while reducing a size of the portable ultrasonic diagnostic apparatus and simultaneously effectively cooling heat generated by circuit boards mounted in a limited space to maintain an appropriate temperature.

### [Disclosure of Invention]

### [Technical Problem]

The present invention provides a portable ultrasonic diagnostic apparatus comprising a heat dissipation structure capable of satisfying necessary waterproof standards while reducing a size of the portable ultrasonic diagnostic apparatus and simultaneously effectively cooling heat generated by circuit boards mounted in a limited space to maintain an appropriate temperature.

### [Technical Solution]

One aspect of the present invention provides a portable ultrasonic diagnostic apparatus comprising a heat dissipation structure according to claim 1.

The heat conducting pipe may be configured as a heat dissipation member in a panel shape including a plurality of heat conducting pads on top surface and bottom surface of one side, which are in close contact with the plurality of circuit boards and absorb the heat generated by the plurality of circuit boards.

One or more of the plurality of heat conducting pads may be configured as heat conducting pads formed of material melted when heat is applied for a certain time in a case that components are mounted on a surface of the circuit board in contact and a hollow portion is present between the components.

The heat dissipation pipe may be bent at least one time to have a structure in which the one side is disposed between the plurality of circuit boards and the other side is disposed in close contact with a bottom surface of the battery accommodating space.

The heat conducting pipe may further include one or more sub heat conducting pipes when the number of the plurality of circuit boards is three or more.

The vent may include an air inlet which suctions cool outside air in the battery accommodating space of the external housing and an air outlet which discharges air inside the battery accommodating space of the external housing to the outside.

The heat dissipation structure may include a cooling fan which forcibly circulates the cool outside air which flows into the battery accommodating space of the external housing toward the air outlet.

The heat dissipation structure may include a heat dissipation plate which is in close in contact with a bottom surface of the other side of the heat conducting pipe and absorbs heat transferred to the other side of the heat conducting pipe.

The heat dissipation plate may include a plurality of heat dissipation cells with cross sections forming quadrangular hollow portions.

The external housing may include a waterproof partition wall which prevents water from flowing into the circuit potion accommodating space, between the circuit portion accommodating space and the battery accommodating space.

### [Advantageous Effects]

As described above, according to embodiments of the present invention, there is provided an effect of reducing a size of the external housing by configuring the circuit portion by arranging the plurality of circuit boards in a stacking structure instead of a single circuit board for forming a conventional circuit portion.

Also, there is an effect of providing a heat dissipation structure of a portable ultrasonic diagnostic apparatus, which includes a heat conducting pipe and an air blowing fan to externally discharge heat generated between circuit boards mounted in a limited internal space of the external housing of the portable ultrasonic diagnostic apparatus, thereby effectively cooling heat generated inside the external housing to maintain an appropriate temperature.

In addition, due to a heat dissipation plate including a plurality of heat dissipation cells with cross sections forming quadrangular hollow portions and formed in parallel, there is provided an effect of increasing an area for absorbing heat transferred to another side of a heat conducting pipe and simultaneously circulating air flowing in from the outside to increase a heat dissipation effect.

Additionally, a waterproof partition wall through which a water-sealed heat conducting pipe passes is provided between a circuit portion accommodating space and a battery accommodating space for accommodating a portable battery, thereby increasing a water-sealing structure inside the circuit portion accommodating space.

### [Brief Description of Drawings]

FIG. 1 is a view of a conventional ultrasonic diagnostic apparatus of an ultrasonic diagnostic system.
FIG. 2 is a block diagram schematically illustrating an internal configuration of a portable ultrasonic diagnostic apparatus according to an embodiment of the present invention.
FIG. 3 is a side cross-sectional view schematically illustrating a heat dissipation structure of the portable ultrasonic diagnostic apparatus according to the embodiment of the present invention.
FIG. 4 is a view illustrating a schematic detailed configuration of reference numeral A shown in FIG. 3.
FIG. 5 is a bottom perspective view schematically illustrating a configuration of the portable ultrasonic diagnostic apparatus according to the embodiment of the present invention.
FIG. 6 is a view schematically illustrating a heat dissipation plate shown in FIG. 5.

### [Mode for Invention]

Embodiments of the present invention are provided to more completely explain the present invention to one of ordinary skill in the art. The following embodiments may be modified into various other forms and the scope of the present invention is not limited thereto. The embodiments are provided to make the disclosure full and complete and to completely convey the concept to those skilled in the art.

The terms used herein are to explain particular embodiments but do not limit the present invention. As used herein, singular expressions, unless contextually defined otherwise, may include plural expressions. Also, the terms "comprise" and/or "comprising" are used herein to specify the present of stated shapes, numbers, steps, operations, members, elements, and/or groups thereof but don not preclude the presence or addition of one or more other shapes, numbers, operations, members, elements and/or groups thereof. As used herein, the term "and/or" includes any and all combinations or one of a plurality of associated listed items.

It should be understood that although the terms "first", "second", etc. are used herein to describe various members, areas, layers, and/or portions, these members, areas, layers and/or portions are not limited by these terms. These terms do not mean particular order, top and bottom, or ratings but are used only to distinguish one member, area, or portion from another member, area, or portion. Accordingly, a first member, area, or portion which will be described below may be referred to as a second member, area, or portion without departing from the scope of the present invention.

Hereinafter, the embodiments of the present invention will be described with reference to the drawings which schematically illustrate the embodiments. In the drawings, for example, depending on a manufacturing technology and/or tolerance, modifications of illustrated shapes may be expected. Accordingly, it should be understood that the embodiments of the present invention are not limited to particular shapes in areas shown in the specification and may include, for example, modifications in shape caused during a manufacturing process.

Meanwhile, the present invention relates to a heat dissipation structure of a portable ultrasonic diagnostic apparatus which transmits an ultrasonic signal to a subject and receives an echo signal reflected by the subject.

Also, a heat dissipation structure of a portable ultrasonic diagnostic apparatus according to the embodiment of the present invention may be reduced in an overall size and may effectively dissipate heat generated between adjacent circuit boards outside an outer housing by including a plurality of circuit boards in a stacking structure instead of a conventional single circuit board on which a main circuit portion and a power control portion are mounted.

Hereinafter, exemplary embodiments of the present invention will be described in detail with reference to the attached drawings.

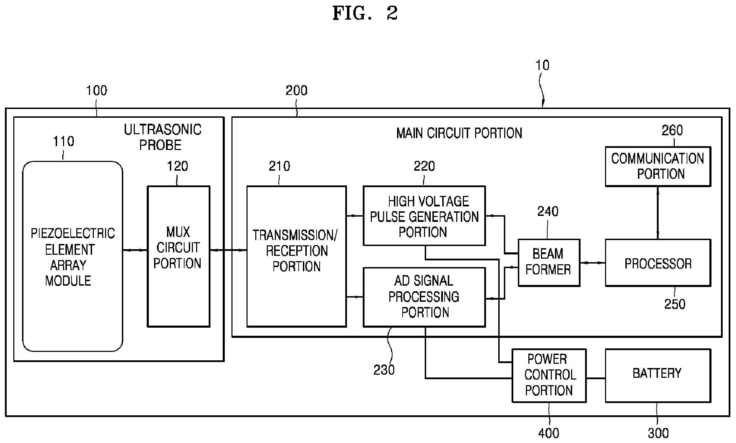

FIG. 2 is a block diagram schematically illustrating an internal configuration of a portable ultrasonic diagnostic apparatus according to an embodiment of the present invention.

As shown in the drawing, a portable ultrasonic diagnostic apparatus 10 according to an embodiment of the present invention includes an ultrasonic probe 100 which generates an ultrasonic signal and receives an echo signal, a main circuit portion 200 which generates an electric pulse for generating the ultrasonic signal and generates an ultrasonic image by analyzing the echo signal received from the ultrasonic probe 100, a portable battery 300 for supplying operation power to the main circuit portion 200, and a power control portion 400 which receives the power from the portable battery 300 and generates and supplies a control voltage needed by the main circuit portion.

Here, the ultrasonic probe 100 includes a piezoelectric element array module 110 and a MUX circuit portion 120 therein to generate the ultrasonic signal and to receive the echo signal. Here, the piezoelectric element array module 110 and the MUX circuit portion 120 include a piezoelectric element, generate ultrasonic waves, and receive echo signals.

Also, the main circuit portion 200 generates the ultrasonic image by receiving and analyzing the echo signal and transmits the ultrasonic image to an external portable display device having a user screen. Also, the power control portion 400 includes a high voltage which drives the ultrasonic probe 100, generates and distributes a voltage necessary for an overall system, and reduces power consumption during an operation to provide a maximal use time while using the battery 300 having limited power as a power source.

In detail, the piezoelectric element array module 110 includes a piezoelectric material. The piezoelectric material perform two functions of generating a pulse of a sound wave by vibrating to transmit the pulse into a human body and receiving and converting a reflected echo into an electrical signal. Currently, as the piezoelectric material, lead zirconate titante (PZT) with highest electroacoustic conversion efficiency is generally used. The piezoelectric element array module 110 is generally configured to arrange a large number of piezoelectric elements such as 64, 128, 192, etc. in an array shape. Here, an electric pulse which drives the piezoelectric element has a range using a high voltage from +100V to -100V and is referred to as an ultrasound transducer.

The MUX circuit portion 120 reduces the number of signal pins. The MUX circuit portion 120 adjusts the number of signals lines between the piezoelectric element array module 110 and a transmission/reception portion 210.

That is, since all the elements in the piezoelectric element array module 110 are not used at the same time and some elements in a position for collecting ultrasonic echo data are used while transmitting an ultrasonic wave and receiving an echo, the elements are electrically selected and connected to the transmission/reception portion 210.

As described above, although the number of piezoelectric elements of the piezoelectric element array module 110 is generally a large number such as 64, 128, 192, etc., when the MUX circuit portion 120 is used as follows, the number of signal lines becomes notably reduced.

Meanwhile, the main circuit portion 200 may control to generate ultrasonic waves to the subject, may receive echo signals received from the piezoelectric element array module 110, may analyze and process a difference between the echo signals as the brightness of a dot to generate an ultrasonic image.

In more detail, the main circuit portion 200 includes the transmission/reception portion 210, a high voltage pulse generation portion 220, an analog-digital (AD) signal processing portion 230, a beam former 240, a processor 250, and a communication portion 260.

The transmission/reception portion 210 may transmit a high voltage pulse generated by the high voltage pulse generation portion 220 to the ultrasonic probe 100 or transmit an analog signal received from the ultrasonic probe 100 to the AD signal processing portion 230. That is, the transmission/reception portion 210 is a switch which connects a TX circuit to the piezoelectric element array module 110 when to transmit ultrasonic waves and connects an RX circuit to the piezoelectric element array module 110 when to receive ultrasonic echoes.

The high voltage pulse generation portion 220 generates an electric pulse to be applied to the piezoelectric element array module 110 to generate ultrasonic waves. Since the intensity of an ultrasonic echo signal returning from the subject is very small, the AD signal processing portion 230 amplifies and converts the ultrasonic echo signal into a digital signal.

It is referred to as TX beam forming to allow the high voltage pulse generation portion 220 to generate an appropriate high voltage pulse using a parameter appropriate for the ultrasonic probe 100. For TX beam forming, the beam former 240 delays time of an electric pulse according to a position of the piezoelectric element to focus energy of ultrasonic waves on a point at a certain distance. It is referred to as RX beam forming to receive the digital signal converted by the AD signal processing portion 230 and perform data-conversion of the digital signal in accordance with the ultrasonic probe 100 to transmit the digital signal to the processor 250. For RX beam forming, the beam former 240 time-delays an electric signal output by each of the piezoelectric elements according to the position of the piezoelectric element and a reception time when the ultrasonic echo is received and generates ultrasonic data (scan data) by adding the time-delayed signals.

Also, the beam former 240 generates and transmits an appropriate digital signal to the AD signal processing portion 230 under the control of the processor 250.

The processor 250 controls the beam former 240 to perform beam-forming appropriate for the ultrasonic probe 100, generates an ultrasonic image using data received from the beam former 240, transmits ultrasonic scan data to an external display device by using the communication portion 260, or controls the whole system. Also, compression of the scan data may be performed to reduce a bandwidth of a transmission line used for communication as necessary.

The communication portion 260 is a communication module which transmits and receives data with an external electronic device. The communication module may use a wired or wireless communication method. In the wired communication method, a cable such as a universal serial bus (USB) cable, etc. may be used. The wireless communication method may include Bluetooth, wireless USB, wireless local area network (LAN), wireless fidelity (Wi-Fi), Zigbee, and infrared data association (IrDA).

The communication portion 260 may display the generated ultrasonic image on a display portion of the external display device under the control of the processor 250. Here, the external display device may be one of a personal computer (PC), a tablet type device, a pad type device, personal digital assistants (PDA), etc.

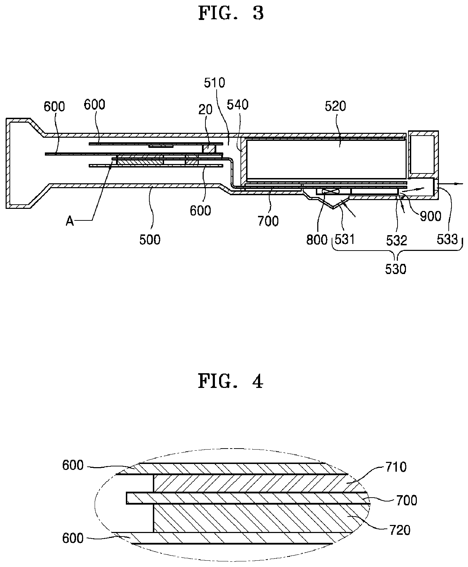

FIG. 3 is a side cross-sectional view schematically illustrating a heat dissipation structure of the portable ultrasonic diagnostic apparatus according to the embodiment of the present invention.

As shown in the drawing, the heat dissipation structure of the portable ultrasonic diagnostic apparatus according to the embodiment of the present invention includes an external housing 500, a plurality of circuit boards 600, and a heat conducting pipe 700.

In more detail, the external housing 500 includes a circuit portion accommodating space 510 in which a circuit board including the main circuit portion and the power control portion is accommodated in one side thereof and a battery accommodating space 520 for accommodating the portable battery therein in another side.

The plurality of circuit boards 600 may be arranged in a vertical stacking structure from a top to a bottom of the circuit portion accommodating space of the external housing 500.

One side of the heat conducting pipe 700 is disposed between the plurality of circuit boards 600 and the other side thereof is disposed in the battery accommodating space 520 to absorb and discharge heat generated by the plurality of circuit boards 600 to the outside air through a vent 530 formed at a place at which the battery accommodating space 520 of the external housing 500 is positioned.

Also, the heat conducting pipe 700 may be doubly bent to have a structure in which one side is disposed between the plurality of circuit boards 600 and the other side is disposed to be in close contact with a bottom surface of the battery accommodating space 520. Due to this, when viewed from a side, heights of the one side and the other side may be different and a step may be present therebetween. However, the heat conducting pipe 700 is not limited thereto and may be bent three times and there may be no step between the heights of the one side and the other side.

Also, not shown in the drawing, the heat dissipation structure of the portable ultrasonic diagnostic apparatus according to the embodiment of the present invention may further include one or more sub heat conducting pipes (not shown) having the same shape as the heat conducting pipe 700.

Also, the heat dissipation structure of the portable ultrasonic diagnostic apparatus according to the embodiment of the present invention may include a waterproof partition wall 540 through which the heat conducting pipe 700 passes between the circuit portion accommodating space 510 and the battery accommodating space 520 for accommodating a portable battery therein to prevent water from flowing into the circuit portion accommodating space 510 which forms the circuit portion of the external housing 500.

In addition, as shown in the drawing, to configure the heat dissipation structure of the portable ultrasonic diagnostic apparatus according to the embodiment of the present invention, the vent 530 may be formed at a place at which the battery accommodating space 520 of the external housing 500 is positioned.

Here, the vent 530 may include an air inlet 531 formed in a center portion of the bottom surface of the battery accommodating space 520 of the external housing 500, a first air outlet 52 formed on an edge of another side of the bottom surface of the battery accommodating space 520 of the external housing 500, and a second air outlet 533 formed on another surface of the external housing 500. However, the numbers and positions of the air inlet and the air outlets are not limited thereto and one or more air inlets and air outlets may be included to increase heat dissipating efficiency.

Meanwhile, in the embodiment of the present invention, a connector 20 which electrically connects circuit boards adjacently stacked may be included.

Also, a plurality of supporters (not shown) may be installed on an edge of the circuit boards to support and fix the circuit boards adjacently stacked and the plurality of circuit boards 600 may be stably fixed in the external housing 500 using fastening means such as bolts and screws.

FIG. 4 is a view illustrating a schematic detailed configuration of reference numeral A shown in FIG. 3.

Referring to FIG. 4, the heat conducting pipe 700 which forms the heat dissipation structure of the portable ultrasonic diagnostic apparatus according to the embodiment of the present invention shown in FIG. 3 will be described in detail as follows.

The heat conducting pipe 700 may include a plurality of heat conducting pads 710 and 720 in close contact with the plurality of circuit boards 600 to absorb heat generated from the plurality of circuit boards 600 on top and bottom surfaces of one side thereof and may be formed as a panel shape heat dissipation member with an oval cross section. However, the cross section of the heat dissipation member is not limited thereto and may be one of various figures such as a circle, a quadrangle, etc.

Here, the heat conducting pipe 700 may be embodied as a container in which a small amount of a working fluid is inserted and sealed in a vacuum state. When the heat generated by the plurality of circuit boards 600 is applied to one side of the heat conducting pipe 700, the working fluid evaporates and the evaporating gas moves with calories as much as the applied heat along an empty space in the container and arrives at the other side of the heat conducting pipe 700 with a temperature decreased due to outside air, thereby externally discharging heat condensed due to a difference in temperature.

Also, one or more of the heat conducting pads 710 and 720 may be formed of a PCM type heat conducting pads melted when heat is applied for a certain time when components are mounted on a surface of a circuit board in close contact to absorb heat and a hollow portion exists to prevent a space between the components on the circuit board.

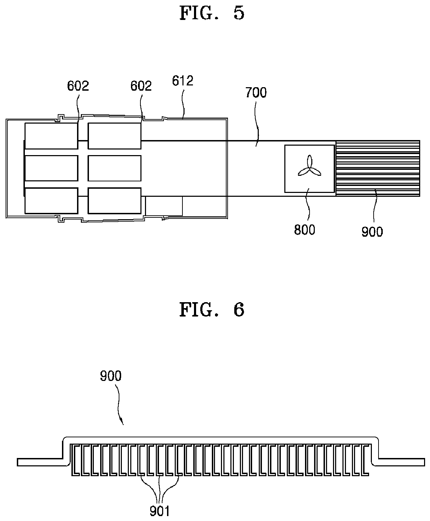

FIG. 5 is a bottom perspective view schematically illustrating a configuration of the portable ultrasonic diagnostic apparatus according to the embodiment of the present invention.

As shown in the drawing, the heat dissipation structure of the portable ultrasonic diagnostic apparatus 10 according to the embodiment of the present invention may include a cooling fan 800 which forcibly circulate outside air suctioned in the external housing 500 toward the air outlets 532 and 533.

Here, the cooling fan 800 may be positioned at a place at which the air inlet 531 of the bottom surface of the battery accommodating space 520 of the external housing 500 shown in FIG. 3 and may be formed in a structure in which the outside air vertically flows downward through the air inlet 531 and is circulated toward the air outlets 532 and 533 through a side thereof However, the position and air flowing in/out directions are not limited thereto and may vary according to a device structure.

Meanwhile, the main circuit portion 200 of FIG. 2 which forms the portable ultrasonic diagnostic apparatus 10 applied to the embodiment of the present invention may include a sensor (not shown) capable of sensing a temperature of heat transferred from the one side to the other side of the heat conducting pipe 700 and a circuit capable of driving the cooling fan 800 when the temperature sensed by the sensor becomes a reference temperature value or more to minimize driving of the cooling fan 800, thereby reducing battery power.

Also, as shown in the drawing, the heat dissipation structure according to the embodiment of the present invention may include a heat dissipation plate 900 disposed in contact with the bottom surface of the other side of the heat conducting pipe 700 and including aluminum or copper. However, a raw material of the heat dissipation plate 900 is not limited thereto and various materials may be used considering a heat dissipation effect.

Here, the heat dissipation plate 900 and the cooling fan 800 may be fastened to be in close contact with the bottom surface of the other side of the heat conducting pipe 700 using fastening means such as bolts and screws with small diameters.

FIG. 6 is a view schematically illustrating the heat dissipation plate shown in FIG. 5.

As shown in the drawing, the heat dissipation plate 900 applied to the embodiment of the present invention may be formed in a longitudinal direction of the external housing 500 of FIG. 3 and may include a plurality of heat dissipation cells 901 which are arranged in parallel and have cross sections forming quadrangular hollow portions. However, the heat dissipation cells 901 are not limited thereto and may be arranged in various directions such as a vertical direction and a diagonal direction of the external housing 500, etc. and the cross section of the heat dissipation cells 901 may have various shapes such as a circular shape, an oval shape, etc.

That is, an area for absorbing heat transferred to the other side of the heat conducting pipe 700 shown in FIG. 5 may be increased through the plurality of heat dissipation cells 901 with the cross sections forming the hollow portions.

Also, at the same time, air which flows in from the outside through the cooling fan 800 described with reference to FIG. 5 is forcibly circulated through the hollow portions, thereby quickly discharging heat outward from the external housing 500.

A heat dissipation process according to the heat dissipation structure of the portable ultrasonic diagnostic apparatus according to the embodiment of the present invention will be described in detail with reference to FIGS. 3 to 6 described above as follows.

First, primarily, the heat generated by the plurality of circuit boards 600 (refer to FIG. 3) may be quickly conducted by the heat conducting pipe 700 (refer to FIG. 4) with one side disposed between the plurality of circuit boards 600 (refer to FIG. 4) and the heat conducting pads 710 and 720 (refer to FIG. 4) and may be transferred to places where the vent 530 (refer to FIG. 3), the cooling fan 800 (refer to FIG. 3), and the heat dissipation plate 900 (refer to FIG. 3) are present.

Also, secondarily, the heat dissipation plate 900 (refer to FIG. 3) formed of aluminum or copper may effectively distribute the heat transferred by the heat conducting pipe 700 (refer to FIG. 3) using the plurality of heat dissipation cells 901 (refer to FIG. 6) to increase heat dissipation efficiency.

Lastly, the heat distributed to the heat conducting pipe 700 (refer to FIG. 5) and the heat dissipation plate 900 (refer to FIG. 5) passing through the process described above may be forcibly circulated by the cooling fan 800 (refer to FIG. 5) and discharged outside the external housing 500 (refer to FIG. 3).

The present invention provides an effect of reducing a size of an external housing by configuring a circuit portion by arranging a plurality of circuit boards in a stacking structure instead of a single circuit board for forming a conventional circuit portion.

Also, there is an effect of providing a heat dissipation structure of a portable ultrasonic diagnostic apparatus, which includes a heat conducting pipe and an air blowing fan to externally discharge heat generated between circuit boards mounted in a limited internal space of the external housing of the portable ultrasonic diagnostic apparatus, thereby effectively cooling heat generated inside the external housing to maintain an appropriate temperature.

In addition, according to the present invention, due to a heat dissipation plate including a plurality of heat dissipation cells with cross sections forming quadrangular hollow portions and formed in parallel, there is provided an effect of increasing an area for absorbing heat transferred to another side of a heat conducting pipe and simultaneously circulating air flowing in from the outside to increase a heat dissipation effect.

Additionally, a waterproof partition wall through which a water-sealed heat conducting pipe passes is provided between a circuit portion accommodating space and a battery accommodating space for accommodating a portable battery therein, thereby increasing a water-sealing structure inside the circuit portion accommodating space.

While the present invention has been described in detail, it should be known that the embodiments described above are only exemplary and not limitative and it will be understood that various changes in form and details may be made therein without departing from the scope of the present invention as defined by the following claims.

## Claims

1. A portable ultrasonic diagnostic apparatus comprising a heat dissipation structure comprising:
an external housing (500) which comprises a circuit portion accommodating space (510) accommodating a circuit board inside one side and comprises a battery accommodating portion (520) for accommodating a portable battery therein inside another side;
a plurality of circuit boards (600) arranged in a vertical stacking structure from top to bottom inside the circuit portion accommodating space of the external housing; and
a heat conducting pipe (700) with one side disposed between the plurality of circuit boards and another side disposed in the battery accommodating space to absorb heat generated by the plurality of circuit boards and discharge the heat into the outside air through a vent (530) formed at a place at which the battery accommodating space of the external housing is positioned.

2. The portable ultrasonic diagnostic apparatus of claim 1, wherein the heat conducting pipe is configured as a heat dissipation member in a panel shape comprising a plurality of heat conducting pads (710, 720) on top surface and bottom surface of one side, which are in close contact with the plurality of circuit boards and absorb the heat generated by the plurality of circuit boards.

3. The portable ultrasonic diagnostic apparatus of claim 2, wherein one or more of the plurality of heat conducting pads are configured as heat conducting pads formed of material melted when heat is applied for a certain time in a case that components are mounted on a surface of the circuit board in contact and a hollow portion is present between the components.

4. The portable ultrasonic diagnostic apparatus of claim 2, wherein the heat dissipation pipe is bent at least one time to have a structure in which the one side is disposed between the plurality of circuit boards and the other side is disposed in close contact with a bottom surface of the battery accommodating space.

5. The portable ultrasonic diagnostic apparatus of claim 1, wherein the heat conducting pipe further comprises one or more sub heat conducting pipes when the number of the plurality of circuit boards is three or more.

6. The portable ultrasonic diagnostic apparatus of claim 1, wherein the vent comprises:
an air inlet (531) which suctions cool outside air in the battery accommodating space of the external housing; and
an air outlet (532, 533) which discharges air inside the battery accommodating space of the external housing to the outside.

7. The portable ultrasonic diagnostic apparatus of claim 6, comprising a cooling fan (800) which forcibly circulates the cool outside air which flows into the battery accommodating space of the external housing toward the air outlet.

8. The portable ultrasonic diagnostic apparatus of claim 1, comprising a heat dissipation plate (900) which is in close in contact with a bottom surface of the other side of the heat conducting pipe and absorbs heat transferred to the other side of the heat conducting pipe.

9. The portable ultrasonic diagnostic apparatus of claim 8, wherein the heat dissipation plate comprises a plurality of heat dissipation cells (901) with cross sections forming quadrangular hollow portions.

10. The portable ultrasonic diagnostic apparatus of claim 1, wherein the external housing comprises a waterproof partition wall which prevents water from flowing into the circuit potion accommodating space, between the circuit portion accommodating space and the battery accommodating space.

## Patentansprüche

1. Tragbares Ultraschalldiagnosegerät, umfassend eine Wärmeableitungsstruktur, umfassend:
ein externes Gehäuse (500), das einen Schaltungsabschnittunterbringungsraum (510) umfasst, der eine Leiterplatte innerhalb einer Seite unterbringt und einen Batterieunterbringungsabschnitt (520) umfasst, der eine tragbare Batterie darin innerhalb einer anderen Seite unterbringt;
eine Vielzahl von Leiterplatten (600), die in einer vertikalen Stapelstruktur von oben nach unten innerhalb des Schaltungsabschnittunterbringungsraums des externen Gehäuses angeordnet sind; und
ein wärmeleitendes Rohr (700), wobei eine Seite zwischen der Vielzahl von Leiterplatten angeordnet ist und eine andere Seite in dem Batterieunterbringungsraum angeordnet ist, um die von der Vielzahl von Leiterplatten erzeugte Wärme aufzunehmen und die Wärme durch eine Entlüftung (530) an die Außenluft abzugeben, die an einer Stelle ausgebildet ist, an der der Batterieunterbringungsraum des externen Gehäuses positioniert ist.

2. Tragbares Ultraschall-Diagnosegerät nach Anspruch 1, wobei das wärmeleitende Rohr als Wärmeableitungselement in einer Plattenform konfiguriert ist, die eine Vielzahl von wärmeleitenden Pads (710, 720) auf der oberen und unteren Fläche einer Seite umfasst, die in engem Kontakt mit der Vielzahl von Leiterplatten stehen und die durch die Vielzahl von Leiterplatten erzeugte Wärme absorbieren.

3. Tragbares Ultraschall-Diagnosegerät nach Anspruch 2, wobei eines oder mehrere der Vielzahl von wärmeleitenden Pads als wärmeleitende Pads konfiguriert sind, die aus geschmolzenem Material gebildet sind, wenn Wärme für eine bestimmte Zeit aufgebracht wird, in einem Fall, dass Komponenten auf einer Oberfläche der Leiterplatte in Kontakt befestigt sind und ein hohler Abschnitt zwischen den Komponenten vorhanden ist.

4. Tragbares Ultraschall-Diagnosegerät nach Anspruch 2, wobei das Wärmeableitungsrohr mindestens einmal gebogen ist, um eine Struktur zu haben, in der die eine Seite zwischen der Vielzahl von Leiterplatten angeordnet ist und die andere Seite in engem Kontakt mit einer Bodenfläche des Batterieunterbringungsraums angeordnet ist.

5. Tragbares Ultraschall-Diagnosegerät nach Anspruch 1, wobei das wärmeleitende Rohr weiter ein oder mehrere wärmeleitende Unterrohre umfasst, wenn die Anzahl der Vielzahl von Leiterplatten drei oder mehr beträgt.

6. Tragbares Ultraschall-Diagnosegerät nach Anspruch 1, wobei die Entlüftung umfasst: einen Lufteinlass (531), der kühle Außenluft in den Batterieunterbringungsraum des externen Gehäuses saugt; und einen Luftauslass (532, 533), der Luft innerhalb des Batterieunterbringungsraums des externen Gehäuses nach außen ableitet.

7. Tragbares Ultraschall-Diagnosegerät nach Anspruch 6, umfassend einen Kühlventilator (800), der die kühle Außenluft, die in den Batterieunterbringungsraum von dem externen Gehäuse in Richtung Luftauslass strömt, zwangsweise umwälzt.

8. Tragbares Ultraschall-Diagnosegerät nach Anspruch 1, umfassend eine Wärmeableitungsplatte (900), die in engem Kontakt mit einer unteren Fläche der anderen Seite des wärmeleitenden Rohres steht und die auf der anderen Seite des wärmeleitenden Rohres übertragene Wärme absorbiert.

9. Tragbares Ultraschall-Diagnosegerät nach Anspruch 8, wobei die Wärmeableitplatte eine Vielzahl von Wärmeableitungszellen (901) mit Querschnitten umfasst, die viereckige Hohlabschnitte bilden.

10. Tragbares Ultraschall-Diagnosegerät nach Anspruch 1, wobei das externe Gehäuse eine wasserdichte Trennwand umfasst, die verhindert, dass Wasser in den Schaltungsabschnittunterbringungsraum zwischen dem Schaltungsabschnittunterbringungsraum und dem Batterieunterbringungsraum fließt.

## Revendications

1. Appareil de diagnostic à ultrasons portable comprenant une structure de dissipation de chaleur comprenant :
un boîtier externe (500) qui comprend un espace de logement de partie de circuit (510) logeant une carte de circuit à l'intérieur d'un côté et comprend une partie de logement de batterie (520) pour y loger une batterie portable à l'intérieur d'un autre côté ;
une pluralité de cartes de circuit (600) agencées en une structure d'empilement vertical de haut en bas à l'intérieur de l'espace de logement de partie de circuit du boîtier externe ; et
un tuyau conducteur de chaleur (700) avec un côté disposé entre la pluralité de cartes de circuit et un autre côté disposé dans l'espace de logement de batterie pour absorber de la chaleur générée par la pluralité de cartes de circuit et évacuer la chaleur dans l'air extérieur via un évent (530) formé à un endroit où l'espace de logement de batterie du boîtier externe est positionné.

2. Appareil de diagnostic à ultrasons portable selon la revendication 1, dans lequel le tuyau conducteur de chaleur est configuré en tant qu'élément de dissipation de chaleur en forme de panneau comprenant une pluralité de tampons conducteurs de chaleur (710, 720) sur une surface supérieure et une surface inférieure d'un côté, qui sont en contact étroit avec la pluralité de cartes de circuit et absorbent la chaleur générée par la pluralité de cartes de circuit.

3. Appareil de diagnostic à ultrasons portable selon la revendication 2, dans lequel un ou plusieurs des différents tampons conducteurs de chaleur sont configurés comme des tampons conducteurs de chaleur formés d'un matériau fondu lorsque de la chaleur est appliquée pendant un certain temps dans le cas où des composants sont montés sur une surface de la carte de circuit en contact et une partie creuse est présente entre les composants.

4. Appareil de diagnostic à ultrasons portable selon la revendication 2, dans lequel le tuyau de dissipation de chaleur est plié au moins une fois pour avoir une structure dans laquelle ledit côté est disposé entre la pluralité de cartes de circuit et ledit autre côté est disposé en contact étroit avec une surface inférieure de l'espace de logement de batterie.

5. Appareil de diagnostic à ultrasons portable selon la revendication 1, dans lequel le tuyau conducteur de chaleur comprend en outre un ou plusieurs sous-tuyaux conducteurs de chaleur lorsque le nombre de la pluralité de cartes de circuit est égal ou supérieur à trois.

6. Appareil de diagnostic à ultrasons portable selon la revendication 1, dans lequel l'évent comprend :
une entrée d'air (531) qui aspire l'air extérieur frais dans l'espace de logement de batterie du boîtier externe ; et
une sortie d'air (532, 533) qui évacue l'air à l'intérieur de l'espace de logement de batterie du boîtier externe vers l'extérieur.

7. Appareil de diagnostic à ultrasons portable selon la revendication 6, comprenant un ventilateur de refroidissement (800) qui fait circuler de force l'air extérieur frais qui circule dans l'espace de logement de batterie du boîtier externe vers la sortie d'air.

8. Appareil de diagnostic à ultrasons portable selon la revendication 1, comprenant une plaque de dissipation de chaleur (900), qui est en contact étroit avec une surface inférieure de l'autre côté du tuyau conducteur de chaleur et absorbe de la chaleur transférée à l'autre côté du tuyau conducteur de chaleur.

9. Appareil de diagnostic par ultrasons portable selon la revendication 8, dans lequel la plaque de dissipation de chaleur comprend une pluralité de cellules de dissipation de chaleur (901) avec des sections transversales formant des parties creuses quadrangulaires.

10. Appareil de diagnostic à ultrasons portable selon la revendication 1, dans lequel le boîtier externe comprend une paroi de séparation étanche qui empêche l'eau de s'écouler dans l'espace de logement de circuit, entre l'espace de logement de circuit et l'espace de logement de batterie.
